# EUROPEAN PATENT APPLICATION

(11) **EP 1 143 009 A1**
(43) Date of publication of application: **10.10.2001**
(21) Application number: 99961297.1
(22) Date of filing: 14.12.1999
(51) Int. Cl.: C12P 21/00

(54) **PROCESS FOR PRODUCING POLYPEPTIDE IN CELL-FREE PROTEIN SYNTHESIS SYSTEM**

(30) Priority: 14.12.1998 JP 35466598
(71) Applicant: Riken, Wako-shi, Saitama 351-0198 (JP)
(72) Inventor: YOKOYAMA, Shigeyuki, Wako-shi, Saitama 351-0198 (JP); KIGAWA, Takanori, Wako-shi, Saitama 351-0198 (JP); YABUKI, Takashi, Wako-shi, Saitama 351-0198 (JP)
(74) Representative: Woods, Geoffrey Corlett
(86) International application number: JP9907016
(87) International publication number: WO0036133

(57) **Abstract**

The present invention relates to a process for producing a polypeptide in a cell-free protein synthesis system using dialysis, which comprises generating the polypeptide via the translation or transcription/translation of a nucleic acid encoding the polypeptide in the cell-free protein synthesis system containing a concentrated cell extract, and recovering the polypeptide. By using this process, a desired polypeptide can be synthesized within a short period of time at a high yield and at a low cost, compared with conventional processes.

## Description

### FIELD OF THE INVENTION

The present invention relates to a process for producing a polypeptide in a cell-free protein synthesis system using dialysis.

### BACKGROUND OF THE INVENTION

A cell-free protein synthesis system is a system in which a protein is synthesized *in vitro* with a cell extract. Cell extracts derived from *E.coli*, wheat germ, or rabbit reticulocyte are mainly used. Since the system can be readily modified, an expression system suitable for a desired protein can be easily constructed. Moreover, a linear DNA created by PCR may be used as a template. This eliminates all the time and effort consuming steps such as ligation to a vector, transformation, incubation, harvesting, and lysis required in the expression system of a living cell, thus allowing expression of a protein readily within a short time. However, since it has a disadvantage of having a low protein yield, the application thereof has been limited.

Since the synthesis system was reported in 1960's, the yield from this system has continued to improve up to the present. *In vitro* reaction method (batchwise) using the cell-free protein synthesis system at the time when the system was developed, yielded a small amount of protein which was only enough to confirm expression thereof with a radioisotope label. A historic improvement of the yield was brought about by the development of a flow method by Spirin et al. (cf. Science 1988, 242, 1162-1164; JP-A-1-503119 (1989)). This method comprises continuous supply of substrates for protein synthesis such as amino acids, ATP, GTP, etc. using a pump, while performing continuous collection of reaction products from a reaction solution drained via an ultrafiltration membrane. By using the flow process, duration of the synthetic reaction, which until then would stop after several hours, was extended up to dozens of hours. This was accompanied by a drastic increase in protein yield; and it became possible to produce 100 µg of protein per ml of the reaction solution. Following this result, the cell-free protein synthesis system became the focus of attention as a system for expression of a protein. Since then, flow methods were reported by several groups (cf. JP-A-4-200390 (1992)). However, the flow method had problems such that substrates were required in large amounts, relative to the resulting protein yield; membranes tended to clog, stopping the reaction; and special devices were required, etc.

Recently, a system has been reported in which synthesis is performed concurrently with supplying a substrate by diffusing it through a dialysis membrane. Kim and Choi (cf. Biotechnol. Prog. 1996, 12, 645-649) developed a chamber with a dialysis membrane spread across the bottom, and performed a protein synthesis soaking this chamber in a substrate solution. Davis et al. (cf. Promega Notes Magazine Number 1996, 56, pp.14-18 (Promega Corporation)) utilized a commercially available dialysis unit, therewith indicating that the duration time for synthetic reaction could be extended using a simpler device than that of the flow method.

Not only alteration of the device, but also modification of cell extract or composition was studied for improvement of the yield. There is a report in which the synthesis rate was improved by concentrating the cell extract to be used for reaction by ultrafiltration centrifugation (cf. Nakano et al., Biosci. Biotech. Biochem., 58, 631-634; Kim et al., Eur. J. Biochem. 1996, 239, 881-886). Moreover, in a cell-free protein synthesis system derived from *E. coli*, changing a combination of phosphoenolpyruvate (PEP) and pyruvate kinase (PK) which was conventionally used as an ATP regenerating system to a combination of creatine phosphate (CP) and creatine kinase (CK), further followed by optimizing the composition of other reaction solutions brought about a yield of several hundreds *µ* g per ml of the reaction solution even in batchwise (Yabuki et al., Journal of Biomolecular NMR 1998, 11:295-306). Herein, both PEP and CP are substrates for regenerating ATP, and both PK and CK are enzymes for converting ADP into ATP. PK and CK require PEP and CP as substrates, respectively.

Up to the present, the maximum yield obtained in a cell-free protein system is 1.2mg/ml in 14 hours (Kim and Choi, *supra).* The synthetic rate was approximately 80 *µ* g/ml/hour, and the yield was approximately 100 *µ* g per ml of the substrate used.

Under the above circumstances, the object of the present invention is to provide the process for producing a polypeptide within a short period of time at high yield and at low cost, compared with conventional processes.

### SUMMARY OF THE INVENTION

The present invention provides a process for producing polypeptide in a cell-free protein synthesis system using dialysis, which comprises generating the polypeptide via the translation or transcription/translation of a nucleic acid encoding the polypeptide in the cell-free protein synthesis system containing a concentrated cell extract; and recovering the polypeptide from the system.

In the present invention, the process comprises shaking or agitating the cell-free protein synthesis system, the cell-free protein synthesis system comprising a reaction solution for polypeptide synthesis containing the concentrated cell extract as an internal dialysis solution and a substrate solution for polypeptide synthesis as an external dialysis solution, and the internal dialysis solution and the external dialysis solution being segregated via a dialysis membrane which permits transfer of substances.

In the present invention, the external dialysis solution of the above-mentioned system may be exchanged with a fresh one when the reaction rate has decreased.

In the present invention, the dialysis membrane may have a molecular weight cut-off more than 10000 dalton, preferably approximately 50000 Da and more.

In the present invention, the concentrated cell extract is a concentrated crude cell extract of a eukaryotic or prokaryotic cell such as *E. coli*, wheat germ, rabbit reticulocyte, mouse L-cell, Ehrlich ascites tumor cell, HeLa cell, Chinese hamster ovarian (CHO) cell, or budding yeast. In one embodiment of the invention, such a concentrated cell extract is a concentrated *E.coli* S30 cell extract. The extract can be obtained by concentration methods such as dialysis, ultrafiltration, polyethylene glycol (PEG) precipitation, and the like. For example, the concentrated *E.coli* 30 cell extract can be obtained by using the *E.coli* S30 extract obtained from an *E.coli* A19 strain (rna, met) with a known method (Zubay et al., Ann. Rev. Genet. 1973, 7, 267-287) (also available from Promega) as an internal dialysis solution and performing dialysis against an external dialysis solution via a dialysis membrane having a 1000-14000 Da molecular weight cut-off in a closed system which can be shaken or agitated. The external dialysis solution used herein may comprise a buffer solution containing potassium acetate, magnesium acetate, and dithiothreitol, and polyethylene glycol or a sucrose/epichlorohydrin water-soluble synthetic copolymer (e.g. Ficoll™ made by SIGMA).

In the present invention, the above-mentioned system may comprise a combination of creatine kinase and creatine phosphate as an ATP regenerating system.

The present invention further provides for a process for producing a polypeptide in a cell-free protein synthesis system using dialysis, which comprises generating the polypeptide via the translation or transcription/translation of a nucleic acid encoding the polypeptide in the cell-free protein synthesis system containing a cell extract derived from *E.coli* and a combination of creatine kinase and creatine phosphate as an ATP regenerating system; and recovering the polypeptide. The cell extract from *E. coli* may be either concentrated or unconcentrated. In one embodiment of the present invention, the above-mentioned cell extract is an *E.coli* S30 cell extract.

The term "cell-free protein synthesis system" as used herein includes a cell-free translation system which reads mRNA information and synthesizes a polypeptide on ribosomes; and both a cell-free transcription system which synthesizes RNA using DNA as a template, and the cell-free translation system.

The term "concentrated cell extract" as used herein means the concentrated crude extract of eukaryotic or prokaryotic cells containing components such as ribosomes and tRNA required for protein synthesis, the extract being concentrated by a known concentration method such as dialysis, ultrafiltration, PEG precipitation (H. Nakano et al., Journal of Biotechnology 1996, 46, 275-282) or a newly found method; and said extract contains components of a translation system or a translation/transcription system involved in *in vivo* protein synthesis. The term "concentration" as used herein means an increase in protein concentration in the extract where a total protein concentration is used as an indication.

The term "polypeptide" as used herein includes polypeptides with any molecular weight constituted by a plurality of amino acid residues, that is, any polypeptide from a low molecular weight (small peptide) to a high molecular weight (large peptide including a protein) are included.

The term "nucleic acid" as used herein indicates any of RNA, mRNA, DNA, or cDNA.

### BRIEF DESCRIPTION OF THE DRAWINGS

Fig.1 shows the yield of chloramphenicol acetyltransferase (also referred to as CAT) protein at a time of six hours after starting the reaction in a cell-free protein synthesis system using dialysis, relative to the molecular weight cut-off of the dialysis membrane or to whether the *E. coli* S30 extract used was concentrated or not.
Fig.2 shows the relationship between the fact of whether or not the external dialysis solution has been exchanged, and the yield of CAT protein relative to synthesis time, in the cell-free protein synthesis system using dialysis
Fig. 3 shows the yield of Ras protein relative to synthesis time in the cell-free protein synthesis system using dialysis

### DETAILED DESCRIPTION OF THE INVENTION

The present invention will be described in detail as follows.

We have unexpectedly found that the yield of a polypeptide is significantly increased in a cell-free protein synthesis process using dialysis when a concentrated crude cell extract is contained in an internal dialysis solution, compared to the case where an unconcentrated extract is used. At the same time, we have found that the yield of a polypeptide can be further improved by using a dialysis membrane having a larger molecular weight cut-off, and/or by exchanging an external dialysis solution with a fresh one when reaction rate decreases.

The crude extract can be derived from eukaryotic and prokaryotic cells with high protein-synthetic activity such as bacteria (e.g. *E. coli*), fungi (e.g. budding yeast), wheat germ, rabbit reticulocyte, mouse L-cell, Ehrlich ascites tumor cell, HeLa cell, CHO cell, etc. (Clemens, M.J., Transcription and translation-a practical approach, (1984), pp.231-270, Henes, B.D.; and Higgins, S.J. eds., IRL Press, Oxford). As defined in the above, the crude cell extract comprises components required for protein synthesis such as ribosomes, tRNA, and the like. Preparation of the crude extract can be performed using a method described in Pratt, J.M. et al., Transcription and translation-a practical approach, (1984), pp. 179-209, Henes, B.D.; and Higgins, S.J. eds., IRL Press, Oxford. Specifically, it can be performed by disintegration with French press (Pratt et al., *supra*) or with glass beads (Kim et al., *supra*). The cell extract is preferably an *E.coli* S30 cell extract. The S30 cell extract can be prepared from *E.coli* A19 strain (rna, met) according to a known method, e.g. Pratt et al. (*supra*), or an extract commercially available from Promega or Novagen can be used.

In the present invention, the above-mentioned cell extract is required to be concentrated such that the total protein concentration is increased. Concentration can be performed according to any method such as ultrafiltration (including ultrafiltration centrifugation), dialysis, PEG precipitation, etc. The degree of concentration is usually at least 1.5-fold, preferably at least 2-fold. In the case of an *E.coli* cell extract, the concentration can be performed up to at least 1.5- to 7-fold by ultrafiltration centrifugation and at least 1.5- to 5-fold by PEG precipitation, but it will be difficult to handle when the degree of concentration exceeds 4-fold. In the case of a wheat germ extract, concentration can be performed up to 10-fold by PEG precipitation (Nakano, H. et al., *supra*). In PEG precipitation, a PEG solution is mixed with a cell extract thereby precipitating and collecting proteins and nucleic acids, which are dissolved in a small quantity of a buffer solution to obtain the concentrated cell extract. Concentration using dialysis can be carried out, for example, according to a method described in the Example below. In one method, the concentrated cell extract can be obtained by using the cell extract as an internal dialysis solution and performing dialysis against an external dialysis solution via a dialysis membrane (e.g. having a 1000-14000 molecular weight cut-off) in a closed system which can be shaken or agitated. The external dialysis solution used herein may include a buffer solution containing potassium acetate, magnesium acetate, and dithiothreitol, and a polymeric absorbent such as PEG (e.g. #8000), sucrose/epichlorohydrin water-soluble synthetic copolymer (e.g. Ficoll™ made by SIGMA), or the like. A polymeric absorbent is essential for absorbing water.

A cell-free system using dialysis with *E.coli* S30 extract was first reported by Beckler et al. in 1992 (ASM poster presentation, 1992), followed by a description by Davis et al. (*supra*) on a large-scale dialysis reaction using the same system, however, each of the S30 extracts used therein were unconcentrated. Davis et al. (*supra*) also indicated in Fig.4C the effect of the difference in a molecular weight cut-off of the dialysis membrane on the yield, however, the improvement of the polypeptide yield was small even when the membrane with a larger cut-off is used.

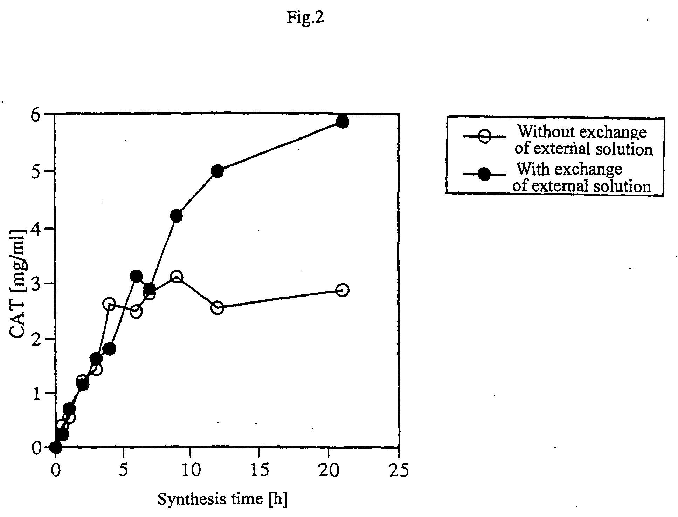

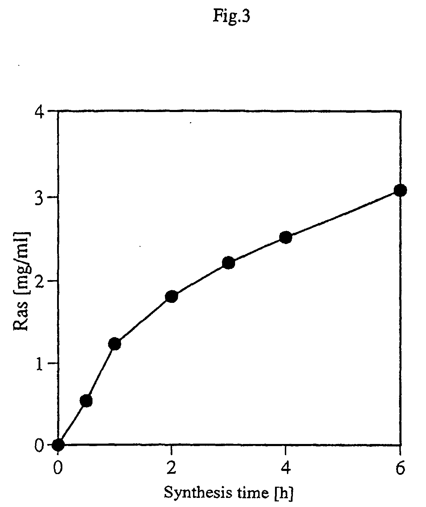

On the contrary, in the process of the present invention, a large increase in yield can be achieved compared to conventional methods, by concentrating the S30 extract and using it as an internal dialysis solution; and/or using a dialysis membrane with a larger molecular weight cut-off; and/or exchanging the external dialysis solution with a fresh one when decrease in the reaction rate is confirmed. Such beneficial effects are shown, for example, in Fig.2 and 3 with respect to time course of the yield of chloramphenicol acetyltransferase (CAT) or Ras proteins in the cell-free system (cf. Example 3 and 4). With respect to the CAT yield, there was an increase to 5mg/ml in a 12-hours-reaction and 6mg/ml in a 21-hours-reaction. While the maximum CAT yield in the cell-free system so far is 1.2mg/ml in a 14-hours-reaction (Kim and Choi, supra), a yield which is at least approx. 4 times greater can be obtained by the present invention.

In the process of the present invention, a dialyser comprising an internal solution and an external solution separated by means of dialysis membrane, which can be shaken and stirred, can be used. Examples of a dialyzer for small-scale reaction include DispoDialyser™(Spectrum) and Slidealyzer™ (Pierce), etc. Examples of a dialyzer for large-scale reaction include a Spectra/Por™ dialysis tube (Spectrum).

The internal dialysis solution (i.e. polypeptide-synthetic reaction solution) in the cell-free protein synthesis system may include DNA or RNA (e.g. mRNA) encoding desired polypeptide, ATP (adenosine 5'-triphosphate), GTP(guanosine 5'-triphosphate), CTP (cytidine 5'-triphosphate), UTP (uridine 5'-triphosphate), buffer, salts, amino acids, RNase inhibitor, antibacterial agent, and where required, RNA polymerase (when DNA is used as a template) and tRNA etc. as well as the concentrated *E.coli* S30 cell extract. Furthermore, a combination of phosphoenolpyruvate and pyruvate kinase or a combination of creatine phosphate and creatine kinase, polyethylene glycol (e.g. #8000), 3', 5'-cAMP, folic acids, an RNase inhibitor, a reducing agent (e.g. dithiothreitol) etc. can be included as a ATP regenerating system. On the other hand, with respect to the external dialysis solution (i.e. substrate solution for polypeptide synthesis), a solution where the cell extract, RNase inhibitor, DNA or RNA, and a RNA polymerase have been removed from the composition of the internal dialysis solution, can be used. For example, a buffer, ATP, GTP, CTP, UTP, salts, amino acids, and antibacterial agents can be included. The concentrations of the added components can be selected appropriately.

With respect to the buffer solution, buffers such as Hepes-KOH, Tris-OAc and the like can be used. Examples of salts include salts of acetic acid (e.g. ammonium salt, magnesium salt etc.), salts of glutamic acid etc., and examples of antibacterial agents include sodium azide, ampicillin, etc. Amino acids include the 20 types of amino acids from which proteins are generally composed. When DNA is used as a template, RNA polymerase is added to the reaction system; for example, a commercially available enzyme such as T7RNA polymerase can be used.

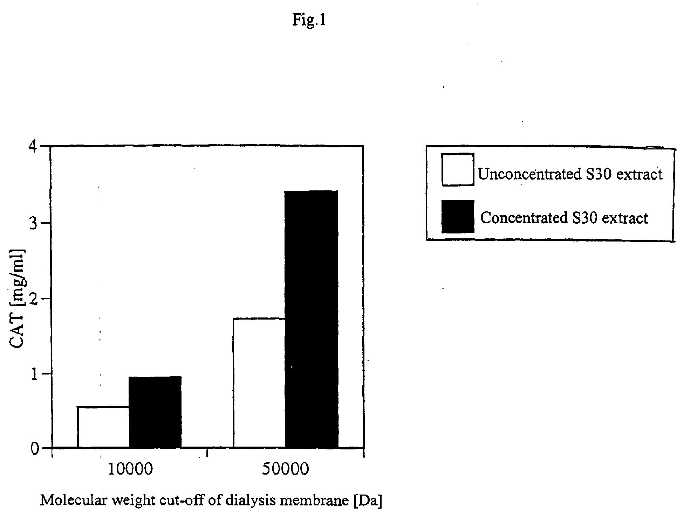

In the present invention, there is the advantage with a cell-free protein synthesis system that even by generating the polypeptide via the translation or transcription/translation of a nucleic acid encoding the polypeptide in the cell-free protein synthesis system containing the *E.coli* cell extract and a combination of creatine kinase and creatine phosphate as the ATP regenerating system; and collecting the polypeptide, the synthesis efficiency goes beyond that of conventional methods. This is clearly seen in the results of Fig.1, and in this case, the cell extract may be concentrated or unconcentrated, preferably concentrated. In this embodiment of the invention, the cell extract is, but is not limited to, the *E.coli* S30 cell extract. The above-mentioned conditions can be applied to other components or the like in the cell-free protein synthesis system.

In the present invention, as defined above, the polypeptide includes any peptide from small peptides to large ones, and known or novel polypeptides are included. DNA or RNA encoding a desired polypeptide can be obtained from eukaryotic or prokaryotic cells or tissues as genomic DNA or mRNA by a well-known method (phenol/chloroform treatment, ethanol precipitation, cesium chloride density gradient centrifugation etc.), or can be synthesized/isolated by cDNA cloning. Alternatively, a sequence of naturally occurring DNA or RNA may be mutated (e.g., substituted, deleted or added) in a usual manner such as site-specific mutagenesis, thereby synthesizing mutants thereof. When an amino acid sequence of a polypeptide or a nucleotide sequence which encodes it, is clarified, it may be chemically synthesized using a DNA synthesizer.

In the practice of the invention, using the above-mentioned dialyzer, the closed system, in which the above-mentioned internal and external dialysis solutions are respectively contained inside and outside of the dialysis membrane through which transfer of substances is permitted depending on the molecular weight cut-off of the membrane, is subjected to shake or agitation (e.g., rotatory agitation), thereby collecting the generated polypeptide of interest from the internal or external dialysis solution. The reaction conditions of temperature and agitation rate etc. can be appropriately set depending on kinds of polypeptides. In protein synthesis, the temperature is generally approximately 25-50°C, preferably 37°C, but in a cell-free protein synthesis system where a cell extract derived from *extreme thermophile* is used, the temperature may be over 50°C. With respect to the shake or agitation rate, a low speed, for example, 100-200rpm can be applied. While observing the generation of the desired polypeptide, the reaction time can be selected appropriately.

In the process of the invention, the polypeptide yield can be further increased by exchanging the external dialysis solution in the above system with a fresh one when the reaction rate decreases; and/or by using the dialysis membrane with a molecular weight cut-off above 10000 Da, preferably approximately 50000 Da and more.

Purification of the generated polypeptide can be performed relatively readily, since the amount and kind of contaminants is remarkably few. Known purification methods can be used alone or in combination appropriately according to the properties of the polypeptide. Purification methods include conventional techniques such as ammonium sulfate or acetone precipitation, acid extraction, anion or cation exchange chromatography, hydrophobic interaction chromatography, affinity chromatography, gel filtration chromatography, HPLC, electrophoresis, chromatofocusing, etc.

Identification and quantification of the generated polypeptide can be performed by assay of activity, immunological measurement, spectroscopic measurement, amino-acid analysis, etc., optionally while comparing to a standard sample.

Embodiments of the invention will be described below by means of illustration, but it is not intended that the scope of the invention is limited to them.

### EXAMPLES

### Example 1: Preparation and concentration of E.coli S30 extract

*E.coli* S30 extract was prepared from the *E.coli* A19 stratin (rna, met) according to the method of Zubay et al. (Annu. Rev. Genet, 1973, 7, 267-187).

15ml of the *E.coli* S30 extract was poured in a dialysis tube, Spectra/Pro2 (having 12000-14000 Da molecular weight cut-off; made by Spectrum), which was sealed with a dialysis tube clamp; and then it was put into a heat seal bag (Yamamoto, Japan) along with 50ml of solution B (in which solution A (10mM Tris-HCl (pH 8.2), 60mM CH₃COOK, 14mM Mg (CH₃COO)₂, and 1mM dithiothreitol (DTT)) was added to 25g of polyethylene glycol #8000 (PEG 8000), giving a total volume of 50ml) which was tight sealed with a heat sealer. This was attached to a rotator and agitated while rotating at 10 rpm at 4°C for 45 min.

The dialysis tube containing the *E.coli* S30 extract was taken out to adjust the position of the clump depending on the decrease in the amount of the solution, followed by dialysis against 500ml of solution A at 4°C for 15 min.

In the above process, the *E.coli* S30 extract having a 2-fold increase in the protein concentration was obtained.

### Example 2 Synthesis of CAT (chloramphenicol acetyltransferase) by cell-free protein synthesis using dialysis

The composition of a protein-synthetic reaction solution (i.e., an internal dialysis solution) was composed of: 55mM Hepes-KOH (pH7.5), 5mM DTT, 1.2mM ATP, 0.8mM each of CTP, GTP and UTP, 80mM creatine phosphate, 250 *µ* g/ml creatine kinase, 4.0% (w/v) PEG8000, 0.64mM 3', 5'-cAMP, 68 *µ* M L-(-)-5-formyl-5,6,7,8-tetrahydrofolic acid, 175 *µ* g/ml *E.coli* tRNA (Boehringer Mannheim), 210mM potassium glutamate, 27.5mM NH₄OAc, 10.7mM Mg(CH₃COO)₂, 1mM each of 20 essential amino acids from which proteins are made, 0.05% NaN₃, 6.7 *µ* g/ml pK7-CAT DNA (CAT expression vector; Kim et al., Eur. J. Biochem. 1996, 239, 881-886), 93 *µ* g/ml T7RNA polymerase, 0.5 unit/ *µ* l RNase inhibitor (Toyobo (Japan)), and 0.3 volumes of the *E.coli* S30 extract from Example 1 or the concentrated *E.coli* S30 extract.

The composition of the substrate solution for protein synthesis (i.e., an external dialysis solution) was constituted by removing the *E.coli* S30 extract, RNase inhibitor, DNA and T7 RNA polymerase from the internal dialysis solution, and adding 4.2mM Mg(CH₃COO)_{2.}

Dispo/Dialyzer CE (having 10000 or 50000 molecular weight cut-off, made by Spectrum) containing 300 µl of the internal dialysis solution was put in a 15ml tube containing 3000 *µ* l of the external dialysis solution, followed by shaking at 160rpm at 37°C in a test tube incubator, to perform a protein synthesis.

Quantification of CAT protein contained in the reaction solution was performed as follows according to Shaw, Methods Enzymol 1975, 735-755. Acetyl coenzyme A and chloramphenicol were used as substrates to acetylate chloramphenicol by CAT, and the resulting reductive coenzyme A was color-developed and quantified with 5,5'-dithiobis-2-nitro benzoic acid (DTNB). CAT activity was quantified from an increased amount of absorbance per unit time at 412nm at 37°C, which was used as an indication to determine the amount of the CAT protein.

The CAT yield at 6 hours after starting the synthesis reaction is shown in Fig.1. In the case of the membrane having 50000 Da molecular weight cut-off, the obtained CAT yield was approximately 2mg/ml with the unconcentrated *E.coli* S30 extract, and approximately 3.5mg/ml with the concentrated one. This figure also indicates that the CAT yield is affected by the magnitude of the molecular weight cut-off of the membrane.

### Example 3 Synthesis of CAT by cell-free protein synthesis using dialysis (where external dialysis solution is exchanged during the reaction)

Using the *E.coli* S30 extract concentrated by the method described in Example 1, the reaction test (with the dialysis membrane having 50000 Da molecular weight cut-off) described in Example 2 was performed; and the resulting CAT yield at the indicated time after start of synthesis is shown in Fig.2. After 6 hours, the CAT synthesis rate was decreased. In the equivalent reaction test, by exchanging the external dialysis solution with a fresh one at the time when the reaction rate was decreased (in this case, at the time of 6 hours), the reaction was continued for extended time, resulting that approximately 5mg/ml and 6mg/ml of CAT were synthesized 12 hours and 21 hours after start of the reaction, respectively (Fig.2).

### Example 4 Synthesis of Ras by the cell-free protein synthesis using dialysis

Using the concentrated *E.coli* S30 extract, the protein synthesis (with a dialysis membrane having 50000 Da molecular weight cut-off) was performed in the same method as in Example 2 except that pK7-Ras (which is an expression vector of Ras protein; Kigawa et al., J. Biomol. NMR 1995, 6, 129-134) instead of pK7-CAT and 1mM [¹⁴C]-leucine (18.5MBq/mmol, Amersham) instead of 1mM leucine were used.

The yield of Ras protein was quantified by the precipitation method using 5% trichloroacetic acid on a filter. As a result, the yield was approximately 3mg/ml 6 hours after start of the reaction.

### Industrial Applicability

Comparison of the present invention with the conventional method based on the results of the above examples shows the following advantages.

### (1) Protein yield

CAT yield in the process of the present invention, 6mg/ml (Example 3) is well above the value of 1.2mg/ml in the conventional method (Kim and Choi; *supra*), which is advantageous for industrial protein production. The protein yield per ml of *E.coli* extract used in the invention is approximately 8mg, which is greater than the value of 3mg by the conventional method. Thus, the protein can be purified concisely with high purity from the reaction solution according to the process of the invention.

### (2) Protein-synthesis rate

The synthesis rate in the process of the present invention is approximately 400 *µ* g/ml/hour, which is far greater than the value of approximately 80 *µ* g/ml by the conventional method. The ability to produce the same yield within a shorter time is advantageous for production of a protein which is prone to be decomposed/denatured.

### (3) Costs required for protein synthesis

The yield per ml of the substrate solution used in the present process is approximately 500 *µ* g, which is far greater than the value of 100 *µ* g/ml by the conventional method. Thus, protein can be produced at a much lower cost by the process of the present invention, compared with the conventional method.

All publications and patent applications cited herein are incorporated herein by reference in their entirety. Furthermore, while the invention can be carried out with various modifications or variations within the scope of the invention described in the attached claims, such modifications or variations are also intended to be included in the present invention.

## Claims

1. A process for producing a polypeptide in a cell-free protein synthesis system using dialysis, which comprises generating the polypeptide via the translation or transcription/translation of a-nucleic acid encoding the polypeptide in the cell-free protein synthesis system containing a concentrated cell extract; and recovering the polypeptide.

2. The process of claim 1 which comprises shaking or agitating the cell-free protein synthesis system, the cell-free protein synthesis system comprising a reaction solution for polypeptide synthesis containing the concentrated cell extract as an internal dialysis solution and a substrate solution for polypeptide synthesis as an external dialysis solution, and the internal dialysis solution and the external dialysis solution being segregated via a dialysis membrane which permits transfer of substances.

3. The process of claim 1 or claim 2 which further comprises exchanging the external dialysis solution with a fresh one when the reaction rate decreases.

4. The process of any one of claims 1 to 3 wherein the dialysis membrane has a molecular weight cut-off more than 10000 Da.

5. The process of any one of claims 1 to 4 wherein the concentrated cell extract is derived from a eukaryotic or prokaryotic cell such as *E. coli*, wheat germ, rabbit reticulocyte, mouse L-cell, Ehrlich ascites tumor cell, HeLa cell, CHO cell, or budding yeast.

6. The process of claim 5 wherein the concentrated cell extract is a concentrated *E.coli* S30 cell extract.

7. The process of claim 5 or claim 6 wherein the concentrated cell extract is obtainable by a concentration method such as dialysis, ultrafiltration, or PEG precipitation.

8. The process of claim 7 wherein the concentrated cell extract is obtainable by using the *E.coli* S30 extract obtained from an *E.coli* A19 strain (rna, met) by a known method as an internal dialysis solution and performing dialysis against an external dialysis solution via a dialysis membrane having 1000-14000 Da molecular weight cut-off, in a closed system which is capable of being shaken or agitated.

9. The process of claim 8 wherein the external dialysis solution comprises a buffer containing potassium acetate, magnesium acetate and dithiothreitol, and polyethylene glycol or a sucrose/epichlorohydrin water-soluble synthetic copolymer.

10. The process of any one of claims 1 to 9 wherein the cell-free protein synthesis system comprises a combination of creatine kinase and creatine phosphate as an ATP regenerating system.

11. A process for producing a polypeptide in a cell-free protein synthesis system using dialysis, which comprises generating the polypeptide via the translation or transcription/translation of a nucleic acid encoding the polypeptide in the cell-free protein synthesis system containing a cell extract derived from *E.coli* and a combination of creatine kinase and creatine phosphate as an ATP regenerating system; and recovering the polypeptide.

12. The process of claim 11 wherein the cell extract is an *E.coli* S30 cell extract.
